**Europäisches Patentamt**

**European Patent Office**

**Office européen des brevets**

(19)

(11) Veröffentlichungsnummer : **0 130 533**
**B1**

(12) **EUROPÄISCHE PATENTSCHRIFT**

(45) Veröffentlichungstag der Patentschrift :
26.03.86

(21) Anmeldenummer : 84107326.5

(22) Anmeldetag : 26.06.84

(51) Int. Cl.⁴ : **C 07 C 69/67**, C 07 C 67/313,
C 07 C 67/54

(54) Verfahren zur Herstellung eines Glyoxylsäureesters.

(30) Priorität : **29.06.83 DE 3323372**

(43) Veröffentlichungstag der Anmeldung :
**09.01.85 Patentblatt 85/02**

(45) Bekanntmachung des Hinweises auf die Patenterteilung : **26.03.86 Patentblatt 86/13**

(84) Benannte Vertragsstaaten :
**AT BE CH DE FR GB IT LI NL SE**

(56) Entgegenhaltungen :
**DE-A- 2 904 775**
**DE-C- 447 838**

(73) Patentinhaber : **HOECHST AKTIENGESELLSCHAFT**
**Postfach 80 03 20**
**D-6230 Frankfurt am Main 80 (DE)**

(72) Erfinder : **Driscoll, Robert Kenneth, Dr.**
**Heilmannstrasse 43**
**D-6000 Frankfurt am Main 50 (DE)**
Erfinder : **Leupold, Ernst Ingo, Dr.**
**Am Zäunefeld 15**
**D-6392 Neu-Anspach (DE)**
Erfinder : **Baltes, Herbert, Dr.**
**Johannesallee 24**
**D-6230 Frankfurt am Main 80 (DE)**
Erfinder : **Ebertz, Wolfgang, Dr.**
**Nelkenweg 12**
**D-5047 Wesseling (DE)**

## Beschreibung

Die vorliegende Erfindung betrifft ein Verfahren zur Herstellung eines Glyoxylsäureesters durch katalytische Oxydehydrierung des entsprechenden Glykolsäureesters in der Gasphase.

Aus der DE-PS-447 838 sowie aus der DE-OS-2 904 775 ist bekannt, daß sich Glykolsäureester an heterogenen Katalysatoren in der Gasphase zu Glyoxylsäureestern oxydehydrieren lassen. Das bei der Reaktion gebildete Wasser und die kondensierbaren Nebenprodukte, unter anderem Alkohole, erschweren die quantitative Abtrennung des jeweiligen Glyoxylsäureesters. Die Anwesenheit des Reaktionswassers bewirkt zumindest eine teilweise Verseifung der Glykolsäureester und Glyoxylsäureester zu Säuren und Alkoholen. Diese Reaktion findet insbesondere in der kondensierten Phase statt. Weiterhin bilden sich unmittelbar bei der Kondensation des Reaktionsgemische das Hydrat, das Halbacetal sowie das Acetal des jeweiligen Glyoxylsäureesters. Für die Spaltung der Hydrate, Halbacetale und Acetale ist bisher kein wirtschaftlich interessanter Weg bekannt geworden. So scheidet beispielsweise die Spaltung dieser Produkte mit stöchiometrischen Mengen $P_2O_5$ wegen der hohen Kosten und wegen der damit verbundenen Abwasserprobleme aus. Ein rein thermische Spaltung der Hydrate, Halbacetale und Acetale ist wegen der hohen Reaktivität der Glyoxylsäureester schwierig.

Es wurde nun gefungen, daß die unerwünschte Bildung der Folgeprodukte Glyoxylsäureesterhydrate, Glyoxylsäureesterhalbacetale, Glyoxylsäureesteracetale, Glyoxylsäure verhindert werden kann, wenn man aus dem gasförmigen Produktgemisch mit einem Schleppmittel das Wasser und den als Nebenprodukt entstandenen Alkohol von Eintritt der Kondensation abtrennt.

Gegenstand der vorliegenden Erfindung ist daher ein Verfahren zur Herstellung eines Glyoxylsäureesters durch katalytische Oxydehydrierung des entsprechenden Glykolsäureesters in der Gasphase, dadurch gekennzeichnet, daß das bei der Oxydehydrierung gebildete gasförmige Reaktionsgemisch nach Zudosierung eines Schleppmittels in den Mittelteil einer Fraktionierkolonne geleitet wird, wobei das Reaktionsgemisch rasch abkühlt und das Reaktionswasser sowie andere Leichtsieder zusammen mit dem Schleppmittel über Kopf abdestillieren und der Glyoxylsäureester als Sumpfprodukt anfällt.

Bei dem erfindungsgemäßen Verfahren werden Glykolsäureester der allgemeinen Formel $HO-CH_2-COOR$ in Dampfform eingesetzt.

Dabei ist R ein Kohlenwasserstoffrest, vorzugsweise ein aliphatischer, geradkettiger oder verzweigter Alkylrest mit 1 bis 8 C-Atomen, insbesondere mit 1 bis 5 C-Atomen.

Die gasförmigen Glykolsäureester werden zusammen mit Sauerstoff oder einem sauerstoffhaltigen Gas, wie Luft, über den Katalysator geleitet. Vorzugsweise verdünnt man mit einem Trägergas, wie Stickstoff oder Edelgasen.

Bei dem erfindungsgemäßen Verfahren setzt man pro Mol Glykolsäureester im allgemeinen folgende Mengen an Zusatzstoffen ein :

Sauerstoff : 0,1 bis 5 Mol, vorzugsweise 0,5 bis 3 Mol
Trägergas : 0 bis 80 Mol, vorzugsweise 30 bis 60 Mol.

Auch außerhalb dieser Grenzen werden noch zufriedenstellende Ergebnisse erzielt.

Als Katalysator für das erfindungsgemäße Verfahren kommt im Prinzip jeder Oxydehydrierungskatalysator infrage. Der Katalysator enthält jedoch vorzugsweise mindestens eines der Element V, Au, Mo, Ag, Cu, Sn, Sb, Bi und P. Jedoch zeigen auch andere Elemente der 3. bis 5. Hauptgruppe eine katalytische Wirkung.

Die genannten Elemente werden entweder in metallischer Form oder in Form ihrer Verbindungen, z. B. als Oxide, Nitrate, Acetate, Acetylacetonate, Oxalate, Citrate oder Halogenide, in die Reaktionszone eingebracht.

Es hat sich als vorteilhaft erwiesen, vor dem Einleiten des Glykolsäureesters in die Reaktionszone ein oxydierendes Gas, insbesondere Sauerstoff oder Luft, oder ein reduzierendes Gas, insbesondere Wasserstoff oder mit Inertgas verdünnten Wasserstoff, bei Temperaturen von 100 bis 800 °C, insbesondere 300 bis 600 °C, über den Katalysator zu leiten.

Die katalytisch aktiven Elemente werden vorzugsweise auf Trägermaterialien aufgebracht. Als Träger eignen sich vor allem Silicate, Aluminiumoxide, Aluminiumsilicate, Bimsstein oder Kohlen. Vorzugsweise verwendet man Silicate, Aluminiumoxide oder Aluminiumsilicate. Besonders vorteilhaft sind Aluminiumsilicate mit einer BET-Oberfläche von weniger als 50 m²/g.

Die Gesamtmenge an Elementen auf dem Träger kann in weiten Grenzen schwanken. Im allgemeinen beträgt sie 0,01 bis 50 Gew.-%, vorzugsweise 0,1 bis 20 Gew.-%, bezogen auf die Gesamtmasse des Trägerkatalysators. Die katalytisch aktiven Komponenten werden zweckmäßig in Form einer Lösung auf den Träger gebracht, dann wird das Lösemittel abgedampft und der Katalysator getrocknet. Als Lösemittel verwendet man im allgemeinen Wasser, Salzsäure, Salpetersäure, Alkalilaugen oder wäßrige Ammoniaklösung, vorzugsweise Wasser und Salzsäure.

Die aktiven Komponenten können jedoch auch ohne Träger eingesetzt werden.

Die Oxydehydrierung wird im allgemeinen bei Temperaturen zwischen 100 und 600 °C, vorzugswei-

2

se zwischen 200 und 400 °C durchgeführt. Die Verweilzeit liegt vorzugsweise zwischen 0,1 und 10 Sekunden, insbesondere jedoch zwischen 0,1 und 1 Sekunde. Auch außerhalb dieser Grenzen erhält man noch befriedigende Ergebnisse.

Die Oxydehydrierung wird bevorzugt bei Normaldruck durchgeführt, jedoch können auch verminderte oder erhöhte Drucke angewendet werden, d. h. 0,01 bis 100 bar.

Im einzelnen geht man so vor, daß der Glykolsäureester und Sauerstoff oder sauerstoffhaltiges Gas, sowie ggf. das Trägergas, aus Dosierungsvorrichtungen in eine Verdampfungszone und die entstandene Gasmischung dann durch ein von außen beheiztes und mit dem Katalysator gefülltes Reaktionsrohr geleitet wird. Es hat sich dabei als vorteilhaft erwiesen, den Sauerstoff oder das sauerstoffhaltige Gas sowie das Trägergas vor der Einleitung in die Verdampfungszone auf die Reaktionstemperatur aufzuheizen.

Bei dem erfindungsgemäßen Verfahren wird das gasförmige Reaktionsgemisch unmittelbar nach Verlassen des Reaktors mit einem Schleppmittel abgeschreckt (gequencht) und direkt in den Mittelteil einer Fraktionierkolonne geleitet. Das Kopfprodukt der Kolonne enthält neben dem Schleppmittel das Wasser und den als Nebenprodukt entstandenen Alkohol sowie noch geringe Mengen anderer Nebenprodukte wie Formaldehyd und Ameisensäureester. Der Sumpf besteht im wesentlichen aus dem Glyoxylsäureester und gegebenenfalls noch unumgesetztem Glykolsäureester ; aus diesem Gemisch kann der Glyoxylsäureester durch eine übliche Destillation abgetrennt werden.

Aus dem Kopfprodukt kann dann das Schleppmittel nach bekannten Methoden, z. B. durch Destillation oder durch Phasentrennung gewonnen und zurückgeführt werden.

Als Schleppmittel eignen sich prinzipiell alle Substanzen, die mit Wasser und Alkoholen Azeotrope bilden. Dabei wird ein mit Wasser nicht-mischbares Schleppmittel bevorzugt, weil dieses dann nach der Kondensation des Kopfproduktes als separate Phase abgetrennt und zurückgeführt werden kann. Der Siedepunkt des Schleppmittels liegt vorzugsweise unter dem Siedepunkt des jeweiligen Glyoxylsäureesters. Zumindest muß jedoch der Siedepunkt des Azeotrops unter dem Siedepunkt des Glyoxylsäuresters liegen.

Geeignete Schleppmittel sind aliphatische und aromatische Kohlenwasserstoffe, die Heteroatome enthalten können, wie $CH_2 Cl_2$, $CHCl_3$, $CCl_4$, n-Pentan, n-Hexan, Cyclohexan, Nonan, Diisopropylether, Methylethylketon, Benzol und Toluol, vor allem n-Pentan und Cyclohexan.

Glyoxylsäureester sind aufgrund ihrer hohen Reaktivität wertvolle Ausgangs- und Zwischenprodukte für eine Reihe von Synthesen pharmazeutisch wirksamer Verbindungen, wie z. B. Allantoin, substituierter Glycine oder Alkaloide (wie z. B. Tetrahydroisochinolin-Alkaloide).

Neuerdings wird der Einsatz von Glyoxylsäureestern zur Herstellung polymerer biologisch abbaubarer Phosphatersatzstoffe für Waschmittel diskutiert.

Die nachfolgenden Beispiele sollen die Erfindung näher erläutern.

Beispiel 1

Ein Aluminiumsilikat mit einer BET-Oberfläche von 1 $m^2$ $g^{-1}$ wird als Träger für den Katalysator verwendet. Der Katalysator enthält 7,8 Gew.- % Ag und 3,7 Gew.- % V. Zur Herstellung des Katalysators löst man 2,0 g Vanadiumpentoxid in 15 ml konzentrierter Salzsäure, tränkt 30 g Katalysatorträger mit dieser Lösung und dampft das Lösungsmittel auf dem Dampfbad ab. In gleicher Weise werden 3,7 g Silbernitrat nach Lösen in 6 ml Wasser aufgebracht. Der Katalysator wird anschließend bei 110 °C getrocknet. 7,5 ml dieses Katalysators werden in einen senkrecht angeordneten Glasreaktor von 150 mm Länge und 20 mm Durchmesser eingefüllt und in einem Gasstrom aus 45 mmol/h Sauerstoff und 2,5 mol/h Stickstoff drei Stunden lang bei 400 °C erhitzt. Der Reaktor wird von außen geheizt und die Temperatur im Inneren wird mit Hilfe eines Thermo-elementes gemessen.

5 ml/h Glykolsäuremethylester (64,8 mmol/h) werden mit einer Kolbenspritze über eine Verdampfungszone in den senkrecht angeordneten Reaktor von oben eingeleitet. Dabei werden der Verdampfungszone gleichzeitig 2,5 mol/h Stickstoff und 58 mmol/h Sauerstoff zugeführt, die beide vorher auf 275 °C aufgeheizt wurden.

Nach einer Anlaufzeit von 1 Stunde zur Einstellung konstanter Betriebsbedingungen wird der Versuch über einen Zeitraum von 4 Stunden fortgeführt.

Die gasförmigen Reaktionsprodukte werden unmittelbar nach Verlassen des Reaktors mit 150 ml/h n-Pentan als Schleppmittel gequencht und in den Mittelteil einer Fraktionierkolonne (Länge : 600 mm, Durchmesser : 55 mm, Füllung : Braunschweiger Wendeln) geleitet. Die Kolonnentemperatur beträgt 70 °C im oberen Teil und 90 °C im unteren Teil. Am Kopf der Kolonne erhält man nach Kühlung ein 2-phasiges Kondensat. Während des 4-stündigen Versuches werden 5,6 g wäßrige Phase produziert mit der Zusammensetzung :

| | |
|---|---|
| $H_2O$ | 70,3 Gew.-% |
| Methanol | 20,8 Gew.-% |
| Formaldehyd | 5,4 Gew.-% |
| Methylformiat | 3,5 Gew.-% |

3

Das Sumpfprodukt besteht überwiegend aus Glyoxylsäuremethylester (17,0 g), entsprechend einer Ausbeute von 74,5 %, bezogen auf eingesetzten Glykolsäuremethylesten.

Beispiel 2

Analog zu Beispiel 1 werden in die dort beschriebene Apparatur 6 ml/h Glykolsäure-n-butylester (46,3 mmol/h), 44,6 mmol/h Sauerstoff und 2,5 mol/h Stickstoff eingeleitet. Der Reaktor ist gefüllt mit 15 ml eines Aluminiumsilicat-Trägerkatalysators, der 8,05 Gew.-% Bi und 1,95 Gew.-% V auf einem Träger wie in Beispiel 1 enthält. Die Reaktionstemperatur beträgt 250 °C.

Die Reaktionsprodukte werden unmittelbar nach Verlassen des Reaktors mit 20 ml/h Cyclohexan als Schleppmittel gequencht und in den Mittelteil der Kolonne geleitet. Die Kolonnentemperatur beträgt 95 °C im oberen Teil und 100 °C im unteren Teil. Am Kopf der Kolonne erhält man nach Kühlung ein 2-phasiges Kondensat. Während des 4-stündigen Versuches werden 2,3 g wäßrige Phase produziert mit der Zusammensetzung :

| | |
|---|---|
| $H_2O$ | 67,8 Gew.-% |
| Butanol | 26,1 Gew.-% |
| Formaldehyd | 6,1 Gew.-% |

Das Sumpfprodukt enthält 10,3 g Glyoxylsäure-n-butylester, entsprechend einer Ausbeute von 42,1 %, bezogen auf eingesetzten Glykolsäure-n-butylester.

## Patentansprüche

1. Verfahren zur Herstellung eines Glyoxylsäureesters durch katalytische Oxydehydrierung des entsprechenden Glykolsäureesters in der Gasphase, dadurch gekennzeichnet, daß das bei der Oxydehydrierung gebildete gasförmige Reaktionsgemisch nach Zudosierung eines Schleppmittels in den Mittelteil einer Fraktionierkolonne geleitet wird, wobei das Reaktionsgemisch rasch abkühlt und das Reaktionswasser sowie andere Leichtsieder zusammen mit dem Schleppmittel über Kopf abdestillieren und der Glyoxylsäureester als Sumpfprodukt anfällt.

2. Verfahren nach Anspruch 1, dadurch gekennzeichnet, daß der Katalysator mindestens eines der Elemente Ag, V, Mo, Cu, Au, Sn, Sb, Bi und P enthält.

3. Verfahren nach Anspruch 1 oder 2, dadurch gekennzeichnet, daß der Siedepunkt des Schleppmittels niedriger als der Siedepunkt des erhaltenen Glyoxylsäureesters ist.

4. Verfahren nach Anspruch 1 oder 2, dadurch gekennzeichnet, daß ein mit Wasser nicht mischbares Schleppmittel verwendet wird.

5. Verfahren nach Anspruch 1 oder 2, dadurch gekennzeichnet, daß n-Pentan oder Cyclohexan als Schleppmittel verwendet wird.

## Claims

1. A process for the preparation of a glyoxylic acid ester by catalytic oxidative dehydrogenation of the corresponding glycolic acid ester in the gas phase, which process is characterized by passing, after metering in an entrainer, the gaseous reaction mixture formed in the oxidative dehydrogenation to the central part of a fractionating column, where-upon the reaction mixture cools rapidly, and the water of reaction and other low boilers distil out together with the entrainer overhead, and the glyoxylic acid ester results as the bottom product.

2. The process as claimed in Claim 1 wherein the catalyst contains at least one of the elements Ag, V, Mo, Cu, Au, Sn, Sb, Bi and P.

3. The process as claimed in Claim 1 or 2, wherein the boiling point of the entrainer is lower than the boiling point of the resulting glyoxylic acid ester.

4. The process as claimed in Claim 1 or 2, wherein an entrainer which is immiscible with water is used.

5. The process as claimed in Claim 1 or 2, wherein n-pentane or cyclohexane is used as the entrainer.

## Revendications

1. Procédé de production d'esters de l'acide glyoxylique par oxydéshydrogénation catalytique de l'ester correspondant de l'acide glycolique en phase gazeuse, procédé caractérisé en ce que l'on fait arriver le mélange réactionnel gazeux formé par la déshydrogénation, après lui avoir ajouté un agent d'entraînement, à la partie moyenne d'une colonne de fractionnement, le mélange se refroidissant

4

rapidement et l'eau formée, ainsi que les autres produits à bas point d'ébullition, étant éliminés par la distillation en tête de la colonne avec l'agent d'entraînement, et on recueille l'ester de l'acide glyoxylique comme produit de fond.

2. Procédé selon la revendication 1, caractérisé en ce que le catalyseur contient un ou plusieurs des éléments Ag, V, Mo, Cu, Au, Sn, Sb, Bi et P.

3. Procédé selon la revendication 1 ou 2 caractérisé en ce que le point d'ébullition de l'agent d'entraînement est inférieur à celui de l'ester de l'acide glyoxylique formé.

4. Procédé selon la revendication 1 ou 2 caractérisé en ce que l'on utilise un agent d'entraînement non miscible à l'eau.

5. Procédé selon la revendication 1 ou 2 caractérisé en ce que l'on utilise du n-pentane ou du cyclohexane comme agent d'entraînement.